# EUROPEAN PATENT SPECIFICATION

(11) **EP 1 219 291 B1**
(45) Date of publication and mention of the grant of the patent: **20.08.2008**
(21) Application number: 01310751.1
(22) Date of filing: 21.12.2001
(51) Int. Cl.: A61K 9/00, A61K 9/14, A61K 9/16, A61K 9/28

(54) **Texture masked particles containing an active ingredient**
Teilchen mit maskiertem Gefüge, einen Wirkstoff enthaltend
Particules avec une texture masquée, comprenant un principe actif

(30) Priority: 21.12.2000 US 745243
(43) Date of publication of application: 03.07.2002
(73) Proprietor: McNEILL-PPC, INC., Skillman, NJ 08858 (US)
(72) Inventor: Parikh, Narendra, Long Valley, NJ 07853 (US); McTeigue, Daniel, N. Wales, PA 19454 (US); Wynn, David W., Abington, PA 19001 (US); Pillai, Ravivaj S., Lansdale, PA 19446 (US)
(74) Representative: Mercer, Christopher Paul

(56) References cited:
- EP-A- 0 458 751
- EP-A- 0 608 850
- WO-A-88/06893
- US-A- 4 675 236
- US-A- 5 455 047
- US-A- 5 681 382

## Description

This invention relates to texture masked particles containing an active ingredient and a polymeric overcoating mixture of a water soluble or water swellable film forming polymer and an anti-grit agent. The coated particles may be used to make chewable tablets or rapidly disintegrating tablets that conveniently may be administered without water.

### Background of the Invention

Pharmaceuticals intended for oral administration are typically provided in solid form as tablets, capsules, pills, lozenges, or granules. Tablets are swallowed whole, chewed in the mouth, or dissolved in the oral cavity. Chewable tablets are typically made from a mixture including active drug particles, and other inactive ingredients (excipients), and are often employed for the administration of pharmaceuticals where it is impractical to provide a tablet for swallowing whole. With chewable tablets, the act of chewing helps to break up the tablet particles as the tablet disintegrates and may increase the rate of absorption by the digestive tract. Chewable tablets are often utilized to improve drug administration in pediatric and geriatric patients.

Certain drug particles have a bitter or otherwise unpleasant taste. In order to make palatable chewable tablets from these, their taste must be masked for example by dispersing or coating the particles with a coating composition as disclosed in, for example, United States Patent Numbers 4,851,226; 5,489,436; 5,529,783; 5,215,755; 5,260,072; 5,460,825; 4,800,087; 5,814,332; and 5,075,114, which are incorporated by reference herein. In general, such efforts have focused on masking the unpleasant taste of the drug by coating the drug particles with, polymers designed to delay dissolution until the drug has cleared the oral cavity. However, after the other ingredients in the tablet matrix dissolve away, the coated drug particles are often left in the mouth with their gritty, sandy texture. This is particularly of concern with rapidly disintegrating dosage forms that are becoming more popular.

Various attempts have been made to enhance the texture of drug particles in order to prevent their adhesion to the oral mucosa upon ingestion. For example, WO88/06893 discloses an oral composition comprised of an active substance and a gelling or swelling agent capable of forming a viscous medium around the particles in an aqueous carrier. Disadvantageously, such compositions must be disintegrated in water to form a liquid suspension before ingestion for purposes of facilitating the ease of quickly swallowing the composition without chewing.

EP0458751A discloses an oral composition comprising a core active composition, a first polymeric coating and a second outer hydrophilic coating. The first polymeric coating comprises a soluble and/or insoluble film forming polymeric material and the second comprises fats, fatty acids, and/or waxes.

It would be desirable to have an oral dosage form that effectively masks the texture or both the taste and the texture of active materials, such as drug particles, during ingestion, which thereby obviates the need for consumption with water; and that also may be chewed.

### Summary of the Invention

According to the present invention there is provided a texture masked particle as defined in appendant claims 1 to 12 and a method of texture masking particles as defined in appendant claims 13 to 16.

In accordance with this invention, texture masked pharmaceutical formulations having an immediate release profile may be made using an overcoating comprising a mixture of a film forming polymer and an anti-grit agent. This texture masking overcoating not only overcomes the gritty texture of the drug particles, but also facilitates ease of swallowing. Moreover, the formulations may conveniently be ingested without the need for water. The overcoated particles of the invention advantageously exhibit sufficient elasticity without the need for added plasticizers to maintain integrity during tableting and prevent release of the drug into the mouth during chewing. Chewable tablets made from these coated particles have excellent taste and yet surprisingly exhibit an immediate release profile.

### Detailed Description of the Invention

As used herein, the term "substantially covers" or "substantially continuous" means that the coating is generally continuous and generally covers the entire surface of the core or underlying layer, so that little to none of the active ingredient or underlying layer is exposed.

The core of the texture masked particle may comprise any one of a number of active ingredients. Suitable active ingredients broadly include pharmaceutically active ingredients, dietary supplements, nutritionals, nutriceuticals, and the like. More specifically these include analgesics, decongestants, expectorants, antitussives, antihistamines, gastrointestinal agents, diuretics, bronchodilators, sleep-inducing agents, vitamins, minerals, anti-infectives, nutrients, and mixtures thereof. One class of preferred active ingredients include nonsteroidal anti-inflammatory drugs (NSAIDs), such as ibuprofen, ketoprofen, flurbiprofen, naproxen, diclofenac. rofecoxib, celecoxib, and aspirin. The active ingredient may alternatively be selected from acetaminophen, pseudoephedrine, phenylpropanolamine, chlorpheniramine, dextromethorphan, diphenhydramine, dimenhydrinate, meclizine, famotidine, loperamide, ranitidine, cimetidine, bisacodyl, psyllium, astemizole, loratadine, desloratadine, fexofenadine, cetirizine, antacids, mixtures thereof and pharmaceutically acceptable salts or metabolites thereof. Most preferably, the active ingredient is selected from the group consisting of acetaminophen, ibuprofen, pseudoephedrine, dextromethorphan, diphenhydramine, chlorpheniramine, loratadine, calcium carbonate, magnesium hydroxide, magnesium carbonate, magnesium oxide, aluminum hydroxide, mixtures thereof, and pharmaceutically acceptable salts thereof.

The core of the particle may comprise pure, crystalline active ingredient, or a mixture of active ingredient with optional ingredients, such as binders, excipients and the like known In the art. The core may be formed using a variety of well known granulation methods, including high sheer wet granulation, spray drying, and fluid bed granulation (including rotary fluid bed granulation). Preferably, the particle core is made by fluid bed granulation. Preferably the average diameter of the core of the particle is from about 80 to about 300 microns.

In one embodiment, the first coating layer, which is comprised of a taste masking agent, substantially covers the core. Examples of suitable taste masking agents include, but are not limited to cellulose acetate, ethylcellulose, poly(ethyl acrylate, methyl methacrylate, trimethylammonioethyl methacrylate chloride), which is commercially available from Rohm Pharma under the tradename, "EUDRAGIT", hydroxypropyl methylcellulose, hydroxypropyl cellulose, hydroxyethyl cellulose, and mixtures thereof.

In another embodiment, the taste masking agent is comprised of a mixture of a) an enteric polymer and b) and insoluble film forming polymer. The enteric polymer may be selected from any one of a variety of known enteric polymers, such as hydroxypropyl methylcellulose phthalate, hydroxypropyl methylcellulose acetate succinate, cellulose acetate phthalate , polyvinylacetate phthalate, and polymethacrylate-based polymers such as poly(methacrylic acid, methyl methacrylate) 1:2, which is commercially available from Rohm Pharma GmbH under the tradename. "EUDRAGIT S" polymers, and poly(methacrylic acid, methyl methacrylate) 1:1, which is commercially available from Rohm Pharma GmbH under the tradename, "EUDRAGIT L" polymers. Combinations of enteric polymers may also be used.

Preferably. the enteric polymer is selected from non-acrylate compounds, specifically hydroxypropyl methylcellulose phthalate, hydroxypropyl methylcellulose acetate succinate, cellulose acetate phthalate, and polyvinylacetate phthalate. Non-acrylates are preferred because acrylate polymers tend to become tacky and agglomerate at high temperature. Cellulose polymers are more heat stable than acrylate polymers. In addition, acrylate polymers are known to have a characteristic, slightly unpleasant taste, whereas cellulose polymers have a more neutral taste profile.

The insoluble film forming polymer may also be selected from a number of known compounds, including cellulose acetate, ethylcellulose, and poly(ethyl acrylate, methyl methacrylate, trimethylammonioethyl methacrylate chloride) 1:2:0.1, which is commercially available from Rohm Pharma under the tradename, "EUDRAGIT RS".. One or more than one insoluble film forming polymer may be used. Preferably, the insoluble film forming polymer is impermeable and does not swell in an aqueous environment. More preferably, the insoluble film forming polymer is selected from cellulose acetate and ethylcellulose.

The weight ratio of enteric polymer to insoluble film forming polymer in the polymeric coating is preferably in the range of about 20:80 to about 80:20, more preferably about 40:60 to about 70:30.

The taste masking agent may be combined with other optional ingredients. In one embodiment, the taste masking agent is combined with one or more non-enteric, water soluble polymers, such as hydroxypropyl cellulose and poly(ethyl acrylate, methyl methacrylate, which is commercially available from Rohm Pharma GmbH under the tradename, "EUDRAGIT NE 30D." When a non-enteric, water soluble polymer is present in the polymeric coating, the level of non-enteric, water soluble polymer is preferably about 10 to about 30% of the polymeric coating.

The taste masking agent may also optionally be combined with a surfactant. Suitable surfactants include both ionic and non-ionic materials from both synthetic and natural origins, including but not limited to lecithin, glyceryl esters, sugar esters, polysorbates, mono and diglycerides of fatty acids, propylene glycol esters, sucrose fatty acid esters, polyoxyethylene derivatives of sorbitan fatty acid esters, and mixtures thereof. Examples of useful polysorbates include sorbitan trioleate, sorbitan monopalmitate, sorbitan monolaurate, propylene glycol monolaurate, glycerol monostearate, diglycerol monostearate, glycerol lactyl-palmitate, Lactic acid derivatives include sodium stearoyl lactylate and calcium stearoyl lactylate. When a surfactant is present in the first coating layer, the level of surfactant is present in an amount, based upon the total weight of the first coating layer, from about 2% to about 10%.

A particularly preferred first layer coating comprises about 53 wt % hydroxypropyl methylcellulose phthalate, about 43 wt % cellulose acetate, and about 4 wt % polysorbate.

The first layer coating is preferably applied to the particle core in the form of a solution using fluidized bed technology, such as Wurster coating or rotor coating. Useful solvents include any of the pharmaceutically suitable organic solvents such as acetone, methanol, ethanol, isopropanol; aqueous solvents such as water; and mixtures thereof. One suitable solvent mixture includes acetone and water at a ratio from about 85:15 to about 95:5.

The thickness of the first layer coating on the core is typically from about 1 micron to about 20 microns, e.g. from about 2 microns to about 15 microns or from about 4 to about 9 microns. The first layer coating may be present in an amount, based upon the total weight of the taste masked particle before the addition of the texture masking overcoating thereto, from about 5 percent to about 50 percent, e.g. from about 15 percent to about 25 percent.

The first layer coating is then overcoated with a texture masking coating layer comprised of a water soluble and/or water swellable film forming polymer and an anti-grit agent- Examples of suitable film forming polymers include, but are not limited to, all pharmaceutically suitable water soluble cellulosic polymers that nonexclusively include hydroxypropyl methylcellulose ("HPMC"), hydroxypropyl cellulose ("HPC"), hydroxyethyl cellulose ("HEC"), and sodium carboxy methyl cellulose ("sodium CMC"); starches; alginates; polyvinyl alcohols; xanthan gums; guar gums; polysaccharides; pectins; gelatins; and mixtures thereof with HPMC being preferred. The anti-grit agents used are polyethylene glycol ("PEG"), polyethylene oxide ("PEO"), and mixtures thereof, with PEG being particularly preferred.

The weight ratio of film-forming polymer to anti-grit agent in the texture masking layer overcoating is 20:80 to 80:20, preferably about 40: 60: to about 60:40, especially about 50:50.

In one embodiment, the texture masking overcoating layer is comprised of about 50 wt % HPMC and about 50 wt % PEG, A particularly preferred HPMC is substitution type 2910 (USP) or 2208 (USP), and has a viscosity of about 6 centipoise in a 2% aqueous solution. A particularly preferred PEG has a molecular weight of about 8000 daltons.

Any of the optional ingredients set forth above for use in the first layer may be used in the same amounts in the texture masking overcoating layer.

The thickness of the texture masking overcoating on the coated core is typically from about 1 to about 20 microns, e.g., from about 2 to about 15 microns or from about 4 microns to about 9 microns. The texture masking overcoating is present in an amount, based upon the weight of the taste masked particle, from about 2 percent to about 40 percent, e.g. from about 3 percent to about 20 percent or about 5 percent to about 10 percent.

The texture masking overcoating may be applied to the coated core via any of the methods set forth above for coating the core with the first taste masking layer. A preferred method for applying the texture masking overcoating is to dissolve the film forming polymer and anti-grit agent in a suitable solvent, then apply the coating solution to the particle core, which is either coated with a taste masking layer or is uncoated, using fluidized bed technology such as Wurster coating or rotor coating. Useful solvents include any of the pharmaceutically suitable organic solvents such as acetone, methanol, ethanol, isopropanol; aqueous solvents such as water; and mixtures thereof. A preferred solvent mixture is ethanol and water. In this embodiment the ratio of ethanol to water in the coating solution is typically from about 10:90 to about 90:10, e.g. from about 50:50 to about 80:20. One skilled in the art may readily appreciate that the coating conditions, such as solution spray rate, drying air temperature and flow rate must be adjusted in order to achieve an equilibrium between the rate of application of the liquid coating solution, and the rate of evaporation of the solvents such that the texture masking coating can be deposited uniformly on the particle to form a complete film without overwetting the particle surface. Details of these methods are well known in the art and set forth in, for example. Lieberman et al., "Pharmaceutical Dosage Forms - Tablets: Volume 3", Chapter 3: Particle Coating Methods (1990), which is incorporated by reference herein.

In another embodiment, the uncoated core layer, i.e. the core layer without a tasternasking coating layer, may be substantially covered with the texture masking overcoating. Any of the optional ingredients set forth above for use in the first layer may be added in the same amounts to the texture masking overcoating. In this embodiment, the texture masking overcoating may be present in an amount, based upon the total weight of core and texture masking overcoating, from about 2 percent to about 40 percent, e.g. from about 3 percent to about 20 weight percent. The texture masking overcoating may be applied to the uncoated core via any of the methods set forth above for applying the tastemasking coating to the core.

In yet another embodiment, the texture masked particle may be manufactured by spray-drying whereby in general the active ingredient is suspended or dissolved, along with the film forming polymer and anti-grit agent and optional other ingredients, in a suitable solvent. Suitable solvents include any of the pharmaceutically suitable organic solvents such as acetone, methanol, ethanol. isopropanol; aqueous solvents such as water; and mixtures thereof. The solution or suspension is then sprayed into a hot drying air stream, resulting in evaporation of the solvent. One skilled in the art may readily appreciate that the spray-drying conditions, such as dryer configuration, spray rate, atomization conditions, drying air temperature and flow rate must be adjusted in order to achieve optimum particle size and morphology. Details of these methods are well known in the art and set forth in, for example, Masters, "Spray Drying Handbook, (1979), which is incorporated by reference herein.

In the embodiment wherein the texture masked particle is produced via spray-drying, the texture masked particle comprises a matrix of active ingredient, film forming polymer, and anti-grit agent such that all of these components may be present at the surface of the particle. The particle will be texture-masked by the presence of the film forming polymer and anti-grit agent at the particle surface in an amount effective for texture masking the active ingredient. The texture masked particles of this embodiment will range in average diameter from about 50 to about 500 microns; e.g. from about 80 to about 400 microns. The weight ratio of film-forming polymer to anti-grit agent in the spray-dried texture masked particle may be in the range of about 10:90 to about 90:10, e.g. about 20:80 to about 80:20, about 60:40 to about 40:60, or about 50:50 to about 50:50. The film forming polymer and the anti-grit agent together are present in an amount, based on the weight of the texture masked spray-dried particle, from about 25 to about 90%, e.g. about 40 to about 80%, or about 50 to about 75%.

Optional ingredients suitable for use in the spray-dried, texture-masked particles include but are not limited to fillers, including water soluble compressible carbohydrates such as sucrose, mannitol, sorbitol, maltitol, xylitol, erythritol, lactose, and mixtures thereof; conventional dry binders including cellulose, cellulosic derivatives, polyvinyl pyrrolidone, starch, modified starch, maltodextrin, and mixtures thereof, and in particular microcrystalline cellulose, maltodextrin, and starch; sweeteners including aspartame, acesulfame potassium, sucralose and saccharin: disintegrants such as microcrystalline cellulose, starch, sodium starch glycolate, crosslinked polyvinylpyrrolidone, crosslinked carboxymethylcellulose; preservatives, flavors, acidulants, antioxidants, glidants, surfactants, and coloring agents.

Tablets comprised of the particles of the present invention may be made by any means known in the art. More specifically, these tablets may be comprised of a mixture of the taste masked and texture masked particles, the texture masked particles, or combinations of the same, along with common tablet excipients known in the art.

Conventional methods for tablet production include direct compression ("dry blending"), dry granulation followed by compression, and wet granulation followed by drying and compression. Other methods include the use of compacting roller technology such as a chilsonator or drop roller, or molding, casting, or extrusion technologies. All of these methods are well known in the art, and are described in detail in, for example, Lachman, et al., "The Theory and Practice of Industrial Pharmacy," Chapter 11, (3^{rd} Ed. 1986), which is incorporated by reference herein. Preferably the tablets may be formed by the direct compression method, which involves directly compacting a blend of the taste masked and texture masked particles, the texture masked particles, or combinations of the same, and any other appropriate optional ingredients. After blending, a pre-determined volume of particles is filled into a die cavity of a rotary tablet press, which continuously rotates as part of a "die table" from the filling position to a compaction position. The particles are compacted between an upper punch and a lower punch to an ejection position, at which the resulting tablet is pushed from the die cavity by the lower punch and guided to an ejection chute by a stationary "take-off' bar.

In embodiments wherein a chewable tablet is desired, the degree of particle compaction is controlled so that the resulting tablets are relatively soft, i.e. they have a hardness of up to about 15 kiloponds per square centimeter (kp/cm²), e.g. from about 1 kp/cm² to about 10 kp/cm² or from about 2 kp/cm² to about 6 kp/cm². "Hardness" Is a term used in the art to describe the diametrical breaking strength as measured by conventional pharmaceutical hardness testing equipment, such as a Schleuniger Hardness Tester. In order to compare values across different size tablets, the breaking strength is normalized for the area of the break (which may be approximated as the tablet diameter times the thickness). This normalized value, expressed in kp/cm², is sometimes referred in the art as tablet tensile strength. A general discussion of tablet hardness testing is found in Leiberman et al.. Pharmaceutical Dosage Forms - Tablets, Volume 2, 2nd ed., Marcel Dekker Inc., 1990, pp. 213 - 217, 327 - 329 (hereinafter "Lieberman").

The active ingredient is present in the chewable tablet in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dose regime, the age and weight of the patient, and other factors must be considered.

The chewable tablet may contain other conventional ingredients such as fillers, including water soluble compressible carbohydrates such as sucrose, mannitol, sorbitol, maltitol, xylitol, erythritol, lactose, and mixtures thereof; conventional dry binders including cellulose, cellulosic derivatives, polyvinyl pyrrolidone, starch, modified starch, and mixtures thereof, and in particular microcrystalline cellulose; sweeteners including aspartame, acesulfame potassium, sucralose and saccharin; disintegrants such as microcrystalline cellulose, starch, sodium starch glycolate; crosslinked polyvinylpyrrolidone, crosslinked carboxymethylcellulose; and lubricants, such as magnesium stearate, stearic acid, talc, and waxes. The chewable tablet may also incorporate pharmaceutically acceptable adjuvants, including for example preservatives, flavors, acidulants, antioxidants, glidants, surfactants, and coloring agents.

Texture masked particles produced in accordance with the present invention advantageously may be used for immediate release applications because the texture masking coating does not retard the dissolution of the active ingredient. Preferably the texture masked particles meet the USP dissolution specifications for the specific active ingredient they contain. In a preferred embodiment for the active ingredient acetaminophen, at least about 70% of the active ingredient is released in 45 minutes from particles tested using USP Dissolution Apparatus II (paddle method) in pH 5.8 phosphate buffer at 75 rpm. In a preferred embodiment for the active ingredient ibuprofen, at least about 70% of the active ingredient is released in 30 minutes from particles tested using USP dissolution apparatus II (paddle method) in pH 7.2 phosphate buffer at 150 rpm.

Specific embodiments of the present invention are illustrated by way of the following examples. This invention is not confined to the specific limitations set forth in these examples, but rather to the scope of the appended claims. Unless otherwise stated, the percentages and ratios given below are by weight.

### Examples

### Example 1. Preparation of comparative chewable tablets

The following ingredients set forth below in Table A were placed in a plastic bag and blended via inverting the bag 100 times:

**Table A: Components of Chewable Particles**

| **Component Name** | **Amount Used** (mg/tablet) |
|---|---|
| Ethylcellulose encapsulated acetaminophen* | 274.7 |
| Aspartame *** | 11.55 |
| Acesulfame Potassium** | 5.78 |
| Citric acid**** | 2.00 |
| Granular mannitol**** | 500 |
| Fumaric acid**** | 20 |
| Microcrystalline cellulose*** | 77 |
| Orange flavor**** | 2 |

| | |
|---|---|
| * comprising, based upon total component weight, a 94.5% acetaminophen core surrounded by a 5.5% ethylcellulose coating layer and available from Eurand America; ** available from Hoechst, GmbH under the tradename, "SUNETT;" *** available from FMC Corporation under the tradename, "AVICEL PH101;" ****These components are readily available and may be commercially purchased from any of the suppliers set forth in the "Handbook of Pharmaceutical Excipients (2^{nd} Ed. 1994). | |

After adding 5.78 mg of magnesium stearate thereto, the resulting mixture was further blended by inverting the bag for an additional 20 times.

The resulting blend was then removed from the bag and compressed on a rotary tablet press at 40 rpm using 19/32" diameter flat faced beveled edge tablet tooling in order to yield tablets having a weight of 898.8 mg, a hardness of 3.1 kp as determined by the Hardness test set forth in Lieberman, and a thickness of 0.19 inches.

### Example 2: Preparation of Texture-Masked Particles

### A. Preparation of Texture-Masking Coating Solution:

A texture masking coating solution was prepared by dispersing equal amount of hydroxypropylmethyl cellulose and polyethylene glycol 800 together with acesulfame potassium (1% of solids) in a solvent comprising 77% ethanol and 23% water so that the solid materials represented 10% of the finished solution. The components of the finished solution are set forth in Table B below:

**Table B: Texture Masking coating Solution Composition**

| **Component Name** | **Amount Used** (g) |
|---|---|
| Ethanol*** | 604.72 |
| Purified water | 177.89 |
| Hydroxypropylmethyl cellulose* | 43.05 |
| Polyethylene glycol 8000*** | 43.05 |
| Acesulfame potassium** | 0.87 |
| Total | 869.58 |

| | |
|---|---|
| * available from Shin Etsu under the tradename, "PHARMACOAT 606;" ** available from Hoechst, GmbH under the tradename, "SUNETT;" ***These components are readily available and may be commercially purchased from any of the suppliers set forth in the "Handbook of Pharmaceutical Excipients (2^{nd} Ed. 1994). | |

### B. Coating the Active Ingredient with Texture Masking Solution

1000 g of the encapsulated acetaminophen starting material from Example 1 were charged into a rotary fluid bed coater (Glatt GPCG-5), The powder bed was mobilized using a rotor speed of 300 rpm and air volume of 0.65 inches of water. The texture masking coating solution was sprayed onto the particles through tangentialiy oriented nozzles at a rate of 30 g per minute Inlet air temperature was 50°C. After all of the solution was sprayed, the resulting texture masked coated particles were dried at a decreased rotor speed of 100 rpm for 5 minutes. The final dried batch weighed 1061 g (97% yield). The level of the texture masking coating materials was 7% by weight of the total finished texture masked and taste masked coated particles. The resulting coated particles had an average diameter of 380 microns with a standard deviation of 70 microns according to a normal distribution model (r²=0.984). 73.8% of the particles had an average diameter between 300 and 425 microns.

### Example 3. Preparation of chewable tablets

Coated particles from Example 2 (7% texture masking overcoating level of HPMC/FEG 8000 on ethylcellulose-coated acetaminophen) were blended with aspartame, acesulfame potassium, citric acid, granular mannitol, fumaric acid, microcrystalline cellulose, and flavor in a plastic bag by inverting 100 times. Magnesium stearate was added, and the mixture was further blended by inverting 20 times. The components of the resulting blend are set forth in Table C below:

**Table C: Components of Chewable Blend**

| **Component Name** | **Amount Used** (mg / tablet) |
|---|---|
| Encapsulated and overcoated acetaminophen (87.9% active)* | 290.7 |
| Aspartame*** | 11.55 |
| Acesulfame Potassium** | 5,78 |
| Citric Acid *** | 2.00 |
| Mannitol *** | 500 |
| Microcrystalline cellulose **** | 77 |
| Fumaric Acid NF*** | 20 |
| Orange flavor*** | 2 |
| Magnesium stearate*** | 5.78 |
| TOTAL | 914.81 |

| | |
|---|---|
| * Prepared in Example 2; ** available from Hoechst, GmbH under the tradename, "SUNETT" *** These components are readily available and may be commercially purchased From any of the suppliers set forth in the "Handbook of Pharmaceutical Excipients (2^{nd} Ed. 1994). **** available from FMC Corporation under the tradename, "AVICEI. PH101;" | |

The resulting blend was compressed on a rotary tablet press at 40 rpm using 19/32" diameter flat faced beveled edge tablet tooling to yield tablets having an average tablet weight of 914.8 mg, a tablet hardness of 3.1 kp as determined by the Hardness test in Lieberman, and a tablet thickness of 0.2 inches. Friability by USP method was 3.3%.

### Example 4: Evaluation of chewable tablets from Examples 1 and 3

The tablets prepared in Examples 1 and 3, respectively, were independently sampled by panelists, who evaluated each respective tablet on the basis of taste, texture, and dissolution.

Both tablets were found to have had a similar taste, with a very slight bitterness detected by most panelists. The tablets from Example 1 were found to have had a perceptible grittiness, which ranged from "slight" to "obvious," and a rough surface. By contrast, the "texture-masked" particles of the present invention produced in accordance with Example 3 were found to have had no grittiness, a smooth texture and a "good melt-away," i.e. the tablet was rapidly cleared from the oral cavity with minimal chewing required.

The tablets from Example 1 and Example 3 were also evaluated for dissolution by USP paddle method (Apparatus II) in a pH 5.8 phosphate buffer at 75 rpm. 100% of the acetaminophen active ingredient was released from the tablets of Example 1 and Example 3 in 45 minutes.

This Example showed that although the texture masked overcoated tablets of the present invention had a flavor similar to that of the prior art tablets, the former were smoother and less gritty. As a result, the texture masked overcoated tablets are more suited for chewable tablet form.

## Claims

1. A texture masked particle comprising:
a) a core containing an active ingredient;
b) a first layer comprised of a taste masking agent that substantially covers the core; and
c) a second layer on the surface of the first layer, the second layer being comprised of
i) a film forming polymer; and
ii) an anti-grit agent selected from polyethylene glycol, polyethylene oxide and mixtures thereof,
wherein the weight ratio of film forming polymer to anti-grit agent in the second layer is in the range of 20:80 to 80:20.

2. The particle of claim 1, wherein the second layer substantially covers the first layer.

3. The particle of claim 1, wherein the active ingredient is a nonsteroidal antiinflammatory drug, acetaminophen, pseudeophedrine, phenylpropanolamine, chlorpheniramine, dextromethorphan, diphenhydramine, dimenhydrinate, mecilzine, famotidine, loperamide, ranitidinc, cimetidine, astemizole, loratadine, desloratadine, fexofenadine, cetirizine, an antacid, a pharmaceutically acceptable salt thereof, a metabolite thereof or a mixture thereof.

4. The particle of claim 1, wherein the taste masking agent is comprised of a mixture of a) an enteric polymer; and b) an insoluble film forming polymer.

5. The particle of claim 4, wherein the enteric polymer is hydroxypropyl methylcellulose phthalate, hydroxpropyl methylcellulose acetate succinate, cellulose acetate phthalate or a mixture thereof.

6. The particle of claim 4, wherein the insoluble film forming polymer is cellulose acetate, ethylcellulose or a mixture thereof.

7. The particle of claim 4, wherein the weight ratio of enteric polymer to insoluble film forming polymer in the first layer is in the range of 20:80 to 80:20.

8. The particle of claim 1 which meets the USP dissolution specification for immediate release dosage forms containing the particular active ingredient.

9. The particle of claim 1 wherein the film forming polymer is hydroxypropyl methylcellulose, hydroxpropyl cellulose, hydroxethyl cellulose, sodium carboxy methyl cellulose, a starch, an alginate, a polyvinyl alcohol, a xanthan gum, a guar gum, a polysaccharide, a pectin, gelatin or a mixture thereof.

10. The particle of claim 1 wherein the weight ratio of film forming polymer to anti-grit agent in the second layer is in the range of about 50:50.

11. A tablet comprised of the particles of any one of claims 1 to 10.

12. A method of texture masking particles comprising an active ingredient, which comprises:
a) applying a substantially continuous first layer over the particles, the first coating layer comprising a taste masking agent; and
b) applying a second layer on the surface of the first layer, the second layer comprising a mixture of 1) a film forming polymer; and 2) an anti-grit agent selected from polyethylene glycol, polyethylene oxide and mixtures thereof, wherein the weight ratio of film forming polymer to anti-grit agent in the second layer is in the range of 20:80 to 80:20.

13. A method of texture masking particles comprising an active ingredient, which comprises:
a) applying a first layer comprised of a taste masking agent over the active ingredient to substantially cover the active ingredient; and
b) applying a coating layer over the first layer, the coating layer comprising a mixture of 1) a film forming polymer; and 2) an anti-grit agent selected from polyethylene glycol, polyethylene oxide and mixtures thereof, wherein the weight ratio of film forming polymer to anti-grit agent in the coating layer is in the range of 20:80 to 80:20.

14. A texture masked particle comprising a matrix which is comprised of:
a) an active ingredient
b) a first layer of a taste masking agent that substantially covers the active ingredient; and
c) a coating layer comprised of:
(i) a film forming polymer, and
ii) an anti-grit agent selected from polyethylene glycol, polyethylene oxide and mixtures thereof,
wherein:
the film forming polymer and anti-grit agent are exposed at the surface of the particle in an amount effective for texture masking the active ingredient, and
the weight ratio of film forming polymer to anti-grit agent is in the range of 20;80 to 80:20.

15. The method of claim 12 or claim 13, wherein the second layer is applied by spray-drying a mixture comprising:
a) a film forming polymer and an anti-grit agent selected from polyethylene glycol, polyethylene oxide and mixtures thereof, which together are present in an amount effective for texture masking the active ingredient; and
b) the active ingredient,
wherein the weight ratio of film forming polymer to anti-grit agent is in the range of 20:80 to 80:20.

## Patentansprüche

1. Textur-maskiertes Teilchen, welches umfasst:
a) einen einen aktiven Bestandteil enthaltenden Kern;
b) eine erste Schicht, die ein Geschmacksmaskierungsagens umfasst, die den Kern im wesentlichen abdeckt; und
c) eine zweite Schicht auf der Oberfläche der ersten Schicht, wobei die zweite Schicht umfasst:
i) ein filmbildendes Polymer; und
ii) ein Antikörnungsagens, ausgewählt aus Polyethylenglykol, Polyethylenoxid und Mischungen derselben;
wobei das Gewichtsverhältnis von filmbildendem Polymer zu Antikörnungsagens in der zweiten Schicht im Bereich von 20:80 bis 80:20 ist.

2. Teilchen nach Anspruch 1, wobei die zweite Schicht die erste Schicht im wesentlichen abdeckt.

3. Teilchen nach Anspruch 1, wobei der aktive Bestandteil ein nicht-steroidaler entzündungshemmender Wirkstoff, Acetaminophen, Pseudoephedrin, Phenylpropanolamin, Chlorpheniramin, Dextromethorphan, Diphenhydramin, Dimenhydrinat, Mecilzin, Famotidin, Loperamid, Ranitidin, Cimetidin, Astemizol, Loratadin, Desloratadin, Fexofenadin, Cetirizin, ein Antacid, ein pharmazeutisch annehmbares Salz derselben, eine Metaboliten derselben oder eine Mischung derselben ist.

4. Teilchen nach Anspruch 1, wobei das Geschmacksmaskierungsagens eine Mischung aus a) einem enterischen Polymer; und b) einem unlöslichen, filmbildenden Polymer umfasst.

5. Teilchen nach Anspruch 4, wobei das enterische Polymer Hydroxypropylmethylcellulosephthalat, Hydroxypropylmethylcelluloseacetatsuccinat, Celluloseacetatphthalat oder eine Mischung derselben ist.

6. Teilchen nach Anspruch 4, wobei das unlösliche, filmbildende Polymer Celluloseacetat, Ethylcellulose oder eine Mischung derselben ist.

7. Teilchen nach Anspruch 4, wobei das Gewichtsverhältnis von enterischem Polymer zu unlöslichem, filmbildenden Polymer in der ersten Schicht im Bereich von 20:80 bis 80:20 ist.

8. Teilchen nach Anspruch 1, welches die USP-Auflösungsspezifikation für sofortige Freisetzungsdosierungsformen erfüllt, die den bestimmten aktiven Bestandteil enthalten.

9. Teilchen nach Anspruch 1, wobei das filmbildende Polymer Hydroxypropylmethylcellulose, Hydroxypropylcellulose, Hydroxyethylcellulose, Natriumcarboxymethylcellulose, eine Stärke, ein Alginat, ein Polyvinylalkohol, ein Xanthangummi, ein Guar-Gummi, ein Polysaccharid, ein Pektin, Gelatine oder eine Mischung derselben ist.

10. Teilchen nach Anspruch 1, wobei das Gewichtsverhältnis von filmbildendem Polymer zu Antikörnungsagens in der zweiten Schicht im Bereich von etwa 50:50 ist.

11. Tablette, die die Teilchen nach einem der Ansprüche 1 bis 10 umfasst.

12. Verfahren zur Texturmaskierung von Teilchen, die einen aktiven Bestandteil umfassen, welches umfasst:
a) Auftragen einer im wesentlichen kontinuierlichen ersten Schicht über die Teilchen, wobei die erste Beschichtungsschicht ein Geschmacksmaskierungsagens umfasst; und
b) Auftragen einer zweiten Schicht auf die Oberfläche der ersten Schicht, wobei die zweite Schicht eine Mischung aus 1) einem filmbildenden Polymer; und 2) einem Antikörnungsagens, ausgewählt aus Polyethylenglykol, Polyethylenoxid und Mischungen derselben, umfasst, wobei das Gewichtsverhältnis von filmbildendem Polymer zu Antikörnungsagens in der zweiten Schicht im Bereich von 20:80 bis 80:20 ist.

13. Verfahren zur Texturmaskierung von Teilchen, die einen aktiven Bestandteil umfassen, welches umfasst:
a) Auftragen einer ersten Schicht, die ein Geschmacksmaskierungsagens umfasst, über den aktiven Bestandteil, um den aktiven Bestandteil im wesentlichen abzudecken; und
b) Auftragen einer Beschichtungsschicht über der ersten Schicht, wobei die Beschichtungsschicht eine Mischung aus 1) einem filmbildenden Polymer; und 2) einem Antikörnungsagens, ausgewählt aus Polyethylenglykol, Polyethylenoxid und Mischungen derselben, umfasst, wobei das Gewichtsverhältnis von filmbildendem Polymer zu Antikörnungsagens in der Beschichtungsschicht im Bereich von 20:80 bis 80:20 liegt.

14. Textur-maskiertes Teilchen, umfassend eine Matrix, die umfasst:
a) einen aktiven Bestandteil,
b) eine erste Schicht eines Geschmacksmaskierungsagens, die den aktiven Bestandteil im wesentlichen abdeckt; und
c) eine Beschichtungsschicht, die umfasst:
i) ein filmbildendes Polymer, und
ii) ein Antikörnungsagens, ausgewählt aus Polyethylenglykol, Polyethylenoxid und Mischungen derselben,
wobei:
das filmbildende Polymer und das Antikörnungsagens an der Oberfläche des Teilchens in einer Menge exponiert sind, die effektiv ist zur Texturmaskierung des aktiven Bestandteils, und wobei das Gewichtsverhältnis von filmbildendem Polymer zu Antikörnungsagens im Bereich von 20:80 bis 80:20 liegt.

15. Verfahren nach Anspruch 12 oder Anspruch 13, wobei die zweite Schicht durch Sprüh-Trocknung einer Mischung aufgetragen wird, die umfasst:
a) ein filmbildendes Polymer und ein Antikörnungsagens, ausgewählt aus Polyethylenglykol, Polyethylenoxid und Mischungen derselben, welche zusammen in einer Menge vorhanden sind, die effektiv ist zur Texturmaskierung des aktiven Bestandteils; und
b) den aktiven Bestandteil,
wobei das Gewichtsverhältnis von filmbildendem Polymer zu Antikönrungsagens im Bereich von 20:80 bis 80:20 liegt.

## Revendications

1. Particule masquée par une texture comprenant :
a) un coeur contenant un principe actif ;
b) une première couche constituée par un agent masquant le goût qui couvre essentiellement le coeur ; et
c) une seconde couche sur la surface de la première couche, la seconde couche étant constituée par
i) un polymère filmogène ; et
ii) un agent anti-abrasion choisi parmi le polyéthylène glycol, l'oxyde de polyéthylène et leurs mélanges ;
dans laquelle le rapport en poids du polymère filmogène à l'agent anti-abrasion dans la seconde couche est dans la plage de 20:80 à 80:20.

2. Particule selon la revendication 1, dans laquelle la seconde couche couvre essentiellement la première couche.

3. Particule selon la revendication 1, dans laquelle le principe actif est un anti-inflammatoire non stéroïdien, un acétaminophène, une pseudophédrine, une phénylpropanolamine, une chlorphéniramine, le dextrométhorphane, une diphénylhydramine, un dimenhydrinate, une mécilzine, une famotidine, un lopéramide, une ranitidine, une cimétidine, un astémizole, une loratidine, une desloratidine, une fexofénadine, une cétirizine, un antacide, un sel pharmaceutiquement acceptable de ceux-ci, un métabolite de ceux-ci ou un mélange de ceux-ci.

4. Particule selon la revendication 1, dans laquelle l'agent masquant le goût est constitué par un mélange de a) un polymère entérique ; et b) un polymère filmogène insoluble.

5. Particule selon la revendication 4, dans laquelle le polymère entérique est le phtalate d'hydroxypropylméthylcellulose, l'acétosuccinate de méthylcellulose, l'acétophtalate de cellulose ou un mélange de ceux-ci.

6. Particule selon la revendication 4, dans laquelle le polymère filmogène insoluble est l'acétocellulose, l'éthylcellulose ou un mélange de ceux-ci.

7. Particule selon la revendication 4, dans laquelle le rapport en poids du polymère entérique au polymère filmogène insoluble dans la première couche est dans la plage de 20:80 à 80:20.

8. Particule selon la revendication 1 qui satisfait la spécification de dissolution USP pour les formes pharmaceutiques à libération immédiate contenant le principe actif particulier.

9. Particule selon la revendication 1, dans laquelle le polymère filmogène est l'hydroxypropylméthylcellulose, l'hydroxypropylcellulose, l'hydroxyéthylcellulose, la carboxyméthylcellulose sodique, un amidon, un alginate, un alcool polyvinylique, une gomme xanthane, une gomme guar, un polysaccharide, une pectine, la gélatine, ou un mélange de ceux-ci.

10. Particule selon la revendication 1, dans laquelle le rapport en poids du polymère filmogène à l'agent anti-abrasion dans la seconde couche est dans la plage d'environ 50:50.

11. Comprimé à base des particules selon l'une quelconque des revendications 1 à 10.

12. Procédé de masquage de texture de particules comprenant un principe actif, qui comprend les étapes consistant à :
a) appliquer une première couche essentiellement continue sur les particules, la première couche d'enrobage comprenant un agent masquant le goût ; et
b) appliquer une seconde couche sur la surface de la première couche, la seconde couche comprenant un mélange de 1) un polymère filmogène ; et 2) un agent anti-abrasion choisi parmi le polyéthylène glycol, l'oxyde de polyéthylène et leurs mélanges, dans lequel le rapport en poids du polymère filmogène à l'agent anti-abrasion dans la seconde couche est dans la plage de 20:80 à 80:20.

13. Procédé de masquage de texture de particules comprenant un principe actif, qui comprend les étapes consistant à :
a) appliquer une première couche constituée par un agent masquant le goût sur le principe actif pour couvrir essentiellement le prinècipe actif ; et
b) appliquer une couche d'enrobage sur la première couche, la couche d'enrobage comprenant un mélange de 1) un polymère filmogène ; et 2) un agent anti-abrasion choisi parmi le polyéthylène glycol, l'oxyde de polyéthylène et leurs mélanges, dans lequel le rapport en poids du polymère filmogène à l'agent anti-abrasion dans la couche d'enrobage est dans la plage de 20:80 à 80:20.

14. Particule masquée par une texture comprenant une matrice qui est constituée par :
a) un principe actif ;
b) une première couche d'agent masquant le goût qui couvre essentiellement le principe actif ; et
c) une couche d'enrobage constituée par :
(i) un polymère filmogène, et
(ii) un agent anti-abrasion choisi parmi le polyéthylène glycol, l'oxyde de polyéthylène et leurs mélanges,
dans laquelle :
◆ le polymère filmogène et l'agent anti-abrasion sont exposés à la surface de la particule en une quantité efficace pour masquer la texture du principe actif, et
◆ le rapport en poids du polymère filmogène à l'agent anti-abrasion est dans la plage de 20:80 à 80:20.

15. Procédé selon la revendication 12 ou la revendication 13, dans lequel la seconde couche est appliquée par séchage par atomisation d'un mélange comprenant :
a) un polymère filmogène et un agent anti-abrasion choisi parmi le polyéthylène glycol, l'oxyde de polyéthylène et leurs mélanges, qui, ensemble, sont présents en une quantité efficace pour masquer la texture du principe actif ; et
b) le principe actif,
dans lequel le rapport en poids du polymère filmogène à l'agent anti-abrasion est dans la plage de 20:80 à 80:20.
